# EUROPEAN PATENT SPECIFICATION

(11) **EP 2 022 513 B1**
(45) Date of publication and mention of the grant of the patent: **20.04.2011**
(21) Application number: 08160455.5
(22) Date of filing: 15.07.2008
(51) Int. Cl.: A61M 1/00

(54) **Silent thoracic drainage device**
Lautloses Thoraxabsauggerät
Dispositif de drainage thoracique silencieux

(30) Priority: 08.08.2007 IT MI20071648
(43) Date of publication of application: 11.02.2009
(73) Proprietor: Eurosets S.r.l., 41036 Medolla (MO) (IT)
(72) Inventor: Fontanili, Paolo, 42015 Correggio RE (IT); Paratelli, Ruggero, 41036 Medolla MO (IT); Balugani, Fabio, 41032 Cavezzo MO (IT)
(74) Representative: Modiano, Micaela Nadia

(56) References cited:
- EP-A- 1 927 369
- US-A- 3 683 913
- US-A- 4 772 277
- US-A- 5 154 712

## Description

The invention relates to a thoracic drainage device.

Devices are known, such as for example disclosed in US-A-4 772 277, which are designed to be connected to the thoracic cavity of a patient after surgery, or a trauma, by means of a tube which conveys to the device exuded liquids and air losses that exit from such cavity, by way of the suction determined by the partial vacuum created within the device proper, or by gravity; these devices are indeed known as thoracic drainage devices.

Known devices are divided into three interconnected contiguous chambers, the first of which is the chamber for collecting the exuded liquids that originate, together with the air losses, from the patient and are intended to accumulate on the bottom of such chamber.

The second chamber is known as sealing chamber; it acts as a one-way valve, since it is capable of receiving the air losses that arrive from the first chamber, preventing their return, and is provided at the bottom with a device for measuring the air losses.

Finally, the third chamber is the chamber for adjusting the vacuum that occurs in the two preceding chambers as a consequence of connection to a source: its presence is necessary in order to ensure constancy of the value of the partial vacuum that is present in said chambers, in view of the fact that the suction source to which they are connected, constituted normally by a vacuum line of a general system, is subject to fluctuations.

Known devices are not free from disadvantageous drawbacks, and it is therefore the aim of the present invention to provide a thoracic drainage device which has improved characteristics from a functional standpoint and from a constructive standpoint.

This aim is achieved by a thoracic drainage device, characterized in that it comprises the features according to the appended claims.

Further characteristics and advantages of the present invention will become better apparent from the description of some preferred but not exclusive embodiments of the device according to the present invention, illustrated by way of non-limiting example in the accompanying drawings, wherein:
Figure 1 is a perspective view of the device according to the invention;
Figure 2 is a sectional view, taken along the line II-II of Figure 1;
Figure 3 is a view of a detail of the device according to the invention;
Figures 4 and 5 are views of a detail of Figure 2 in different functional conditions;
Figures 6 and 7 are views of two details of the device according to the invention;
Figures 8 and 9 are views of the detail of Figure 7 in two different functional conditions;
Figures 10 and 11 are views of two details according to further embodiments.

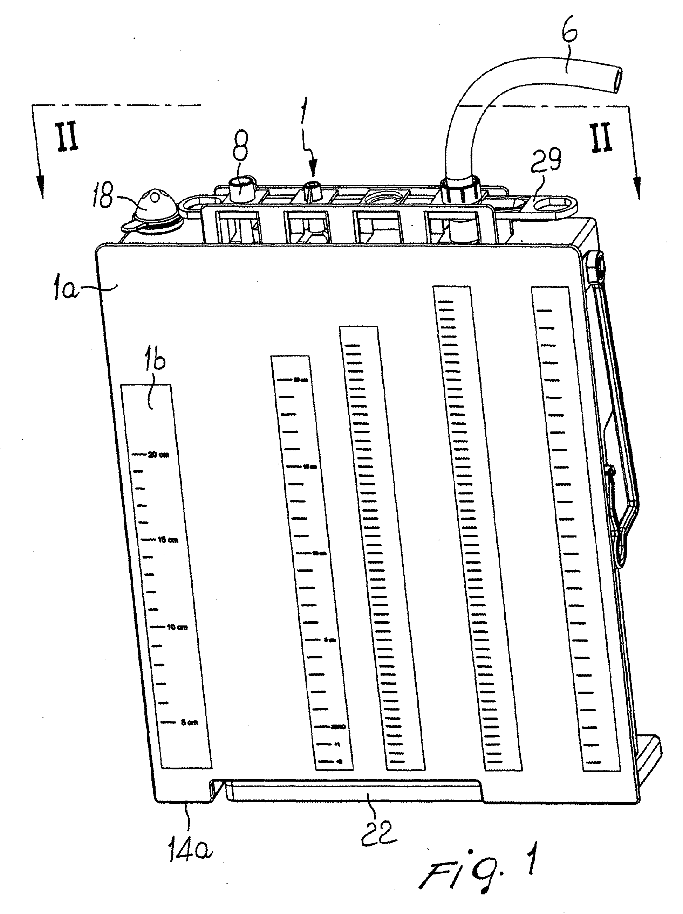

With reference to Figures 1 to 9, the reference numeral 1 generally designates a device, which comprises a first collection chamber 100, delimited by walls 2 and 3 and divided into three interconnected subchambers by partitions 4 and 5; the first collection chamber is designed to be connected by means of a tube 6 to the thoracic cavity of a patient, so as to receive the air losses along the arrow A and the exuded liquids along the arrow B in order to collect the latter at the bottom.

A second chamber 200, delimited by the wall 3 and by the wall formed by portions 7a, 7b, 7c, is designed to be connected by means of a connector 8 to a suction source and is connected by means of a connecting duct 9 to the first chamber 100, so that the same degree of vacuum that determines the suction of the exuded liquids and of the air losses occurs therein.

While, as mentioned, the exuded liquids are collected on the bottom of the first chamber 100, the air losses, following the arrows C and D, pass through the duct 9 and reach the second chamber 200, where they are directed by a partition 10, along the arrow E, toward the bottom in order to penetrate along the arrow F into a measurement device provided thereat, which is generally designated by the reference numeral 11 and is described in detail hereinafter; in output from the device 11 along the arrows G and H, which illustrate the crossing of a tap 12 comprised within the device, the air losses arrive along the arrow I at the connector 8 in order to be evacuated.

A third chamber 300 is delimited by the wall formed by the portions 7a, 7b, 7c and by a wall 13, and the adjustment of the vacuum that occurs in the two previously described chambers occurs therein in a known manner.

In the third chamber there is in fact a water column 14, which is typically 20 centimeters high and through which atmospheric air is made to pass by bubbling and penetrates along the arrow L at the bottom formed by a perforated bubbling partition 15, being conveyed thereto by a duct 16 formed by a partition 17 after entering the device through a plug 18 along the arrow M, to be then evacuated along the arrow N through the connector 8.

Since atmospheric pressure is present at the base of the water column 14, in a portion of space 19 at the top there is necessarily a partial vacuum whose value is equal to the height of the column, and is therefore typically equal to 20 centimeters of water column, and such partial vacuum, shared by the two previously described chambers, is absolutely constant.

Since a wall 1a of the device is provided with transparent regions 1b for viewing the content of the several chambers described above, a detailed description will follow with respect to the third chamber.

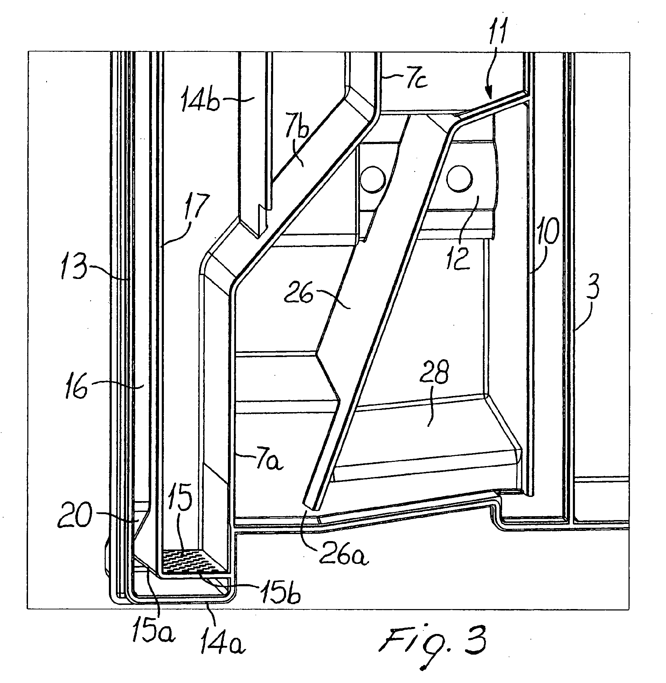

In order to reduce the noisiness of the device, as shown in Figure 3, a particular shaping has been provided for the duct 16, which conveys atmospheric air to the bubbling partition 15 provided monolithically with the structure of the device, by shaping the end portion of the bottom wall of the duct so as to form an inclined plane 20, thereby determining access of the atmospheric air to the bubbling partition 15 at the transverse side 15a thereof, which is much longer than the front side 15b.

One thus obtains an optimum subdivision of the atmospheric air in passing through the holes of the partition 15, with the formation of bubbles of minimum size and thus such as to ensure a good reduction of noisiness, which is directly proportional to the size of the bubbles.

Again with the goal of reducing the noisiness of the device, the sound waves in output have been subdivided by providing the plug 18 with four holes 18a, 18b, 18c, 18d. Moreover, the third chamber 300 of the device is provided with means which are capable of preventing the passage of water, due to intense bubbling of the atmospheric air in the water column 14, through a port 21 which connects the third chamber to the second chamber 200 and to the connector 8 of the suction source. From this standpoint, a base 14a of the device at the column 14 has been arranged so as to be coplanar with respect to the base of a support 22 of the device, thus obtaining the maximum spacing of the free surface of the column 14 from the port 21 without causing unwanted increases in the overall vertical space occupation of the device.

For the same purpose, at the top of the portion of space 19 there is a phase separator, which comprises a baffle 23a and a separator 23b; the two-phase stream determined by bubbling, which comprises water, represented by the black arrow, and atmospheric air, represented by the white arrow, enters through a port 24 the duct which is delimited by the two partitions and ends with a double opening: through a port 25a, the water descends toward the underlying column 14, separating from the current of atmospheric air that passes through a port 25b and immediately reaches, in a perfectly dry condition, the output port 21.

The presence of a partition 14b makes the described separator particularly effective.

The device 11 for measuring the air losses of the patient, which is present at the base of the second sealing chamber, is now described.

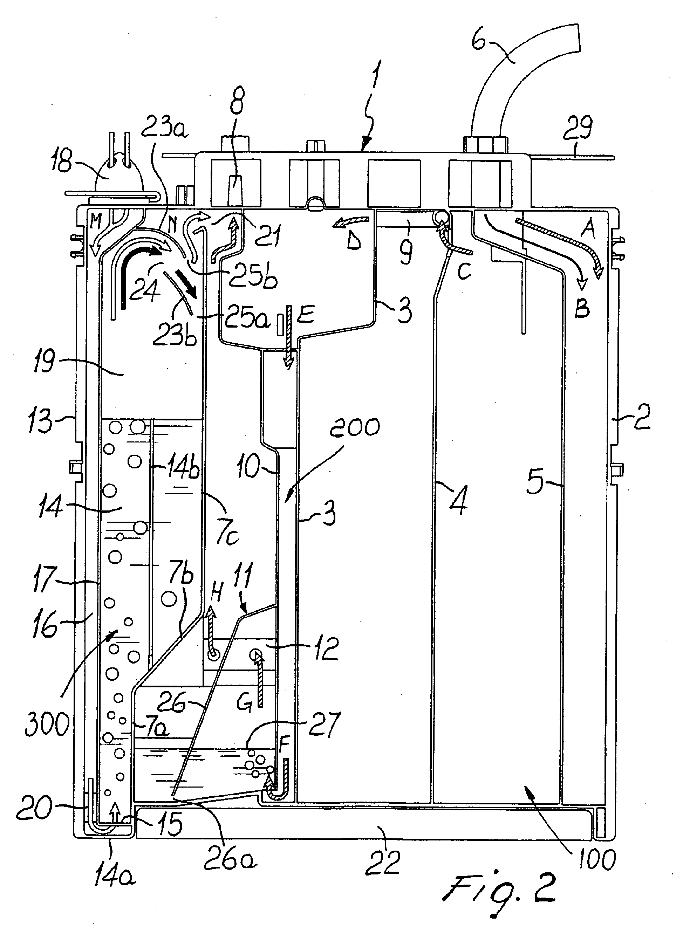

Device 11 comprises a bell 26, which has a passage port 26a at the lower end and is provided with the tap 12; at the base of the bell there is water, which in the normal operating conditions shown in Figure 2, with the tap 12 open and therefore such as to allow the passage of the air losses along the arrows G, H, maintains its free surface at a constant level 27.

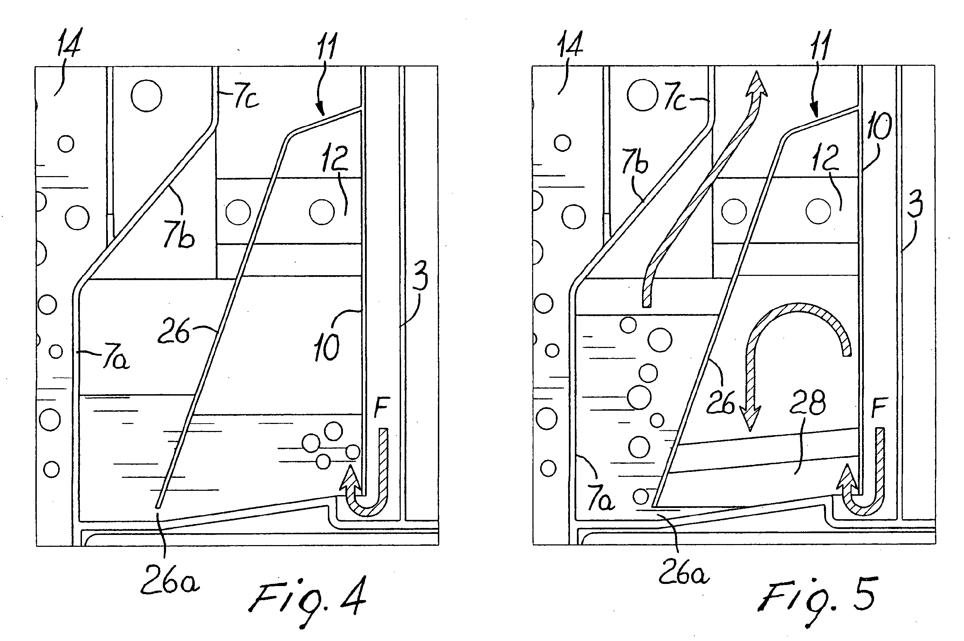

In order to measure the air losses, the tap 12 is closed, and so the air losses accumulate below the bell 26, gradually expelling the water through the port 26a, progressively providing situations of the type shown in Figure 4.

The measurement of the quantity of the losses is deduced from the time taken by the air losses to completely empty the bell of water, reaching the situation of Figure 5, and the importance for the operator of identifying the precise instant of the end of the emptying of the water from the bell is immediately evident.

For this purpose, a plane 28 that lies at the base of the bell has been given such an inclination as to make the region that directly faces the port 26a the lowest one.

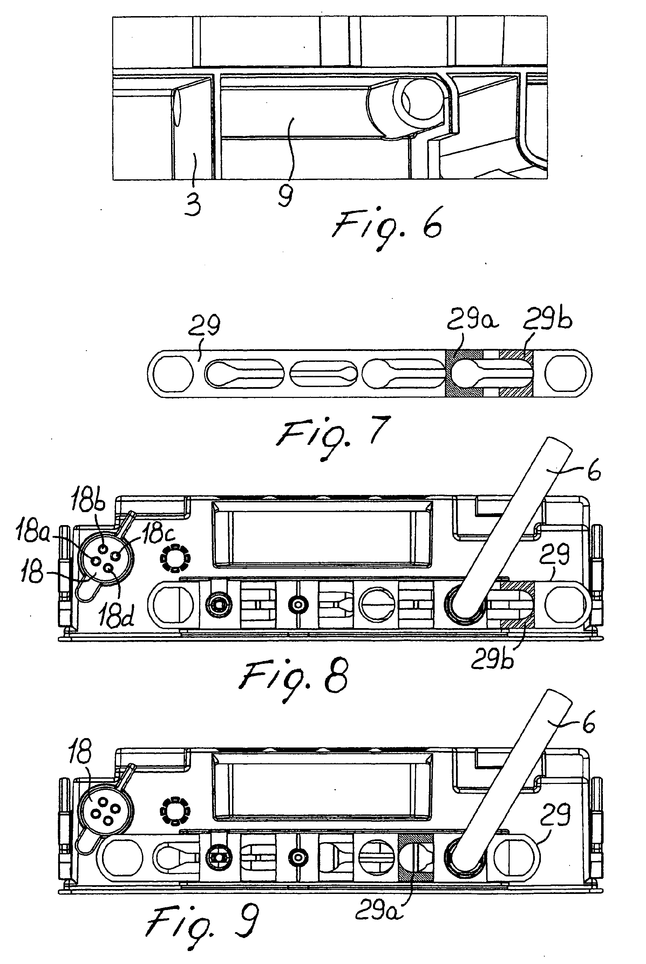

Considering in particular Figure 6, it is seen that the duct 9 for connection between the first chamber 100 and the second chamber 200 of the device is formed monolithically with the structure of the device, following the same design concept that provided monolithically also the bubbling partition 15 and substantially seeks to obtain the entire device with a single mold.

Finally attention is focused on a tube clamping plate 29, which is operated by the operator to obtain different functional conditions between the two extreme positions which are visible respectively in Figures 8 and 9.

In order to provide an indication which is immediately perceivable without any possibility of doubt regarding the assumed position, the plate 29 is provided with two regions 29a, 29b, which have different colorings and are designed to be selectively visible in the two extreme positions: thus, in the position of Figure 8, the region 29b is visible, and in the position of Figure 9 the region 29a is visible.

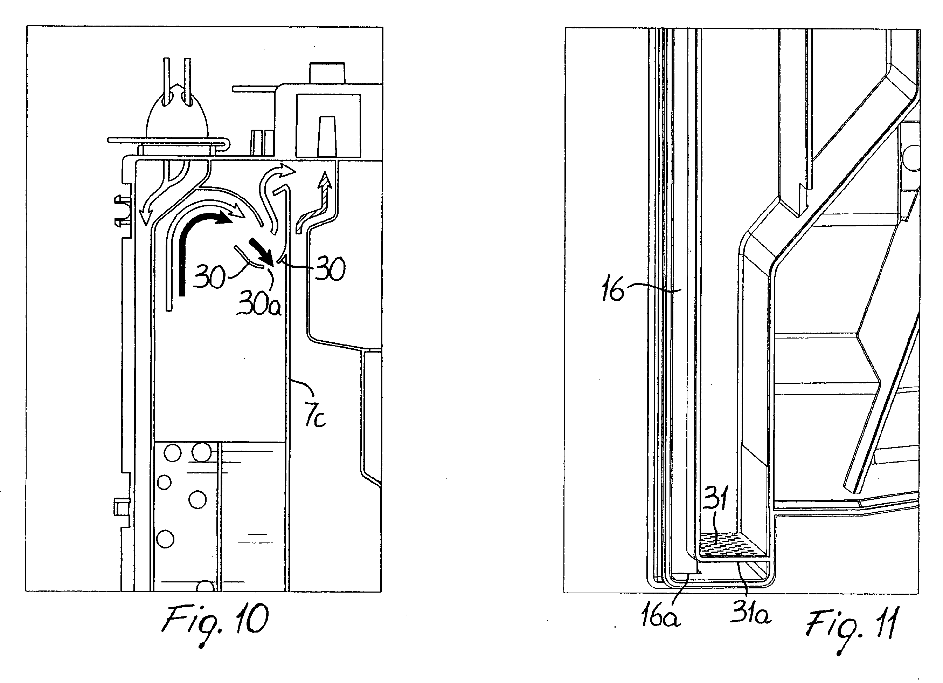

Figure 10 shows a further embodiment of the device according to the invention related to the phase separator described with reference to Figure 2; with respect to the previously described embodiment, the only variation consists in that the separation partition, i.e., the partition that in such embodiment was indicated by the reference numeral 23b, is provided here in the form of a cradle 30, which is connected to the wall 7c and has a port 30a for discharging the water, all the rest remaining unchanged.

Figure 11 shows another embodiment of the device according to the invention, which relates to the bubbling partition and to the methods for feeding atmospheric air thereto which are described with reference to Figure 3.

In this case, the bottom wall of the duct 16 that conveys the atmospheric air to a perforated bubbling partition 31 is flat and extends until it touches at 16a the bottom of the device, and thus the access of the air to the partition 31 occurs by means of a front side 31a; in this solution, the partition 31 has a differentiated calibration of the holes with a diameter which increases progressively as one moves away from the side 31a.

The described invention is susceptible of numerous other modifications and variations, all of which are within the scope of the appended claims; all the details may further be replaced with other technically equivalent elements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A thoracic drainage device (1), comprising, in a monolithic structure, three interconnected contiguous chambers (100,200,300), which are adapted to be placed in partial vacuum by being connected to a source of suction which is capable of creating, within the device, a condition of vacuum which conveys away from the patient the exuded liquids and the air losses, said chambers comprising: a first chamber (100) for collecting the exuded liquids, which is adapted to be connected by means of a tube (6) to the thoracic cavity of a patient, so as to receive said exuded liquids and the air losses that exit from said cavity, a second sealing chamber (200), provided at the bottom with a device (11) for measuring said air losses, a third chamber (300) for adjusting the vacuum that occurs within the two preceding chambers (100,200), comprising a column of water (14) through which atmospheric air is made to pass which enters at the bottom formed by a perforated bubbling partition (15;31) and is conveyed thereat by a duct (16) after entering through an appropriately provided perforated plug (18) in order to be evacuated through a connector (8) which is designed to be connected to the source of suction, said bubbling partition (15;31) comprising a front side (15b;31a), and a transverse side (15a) that is arranged adjacent said duct (16) for conveying atmospheric air to said bubbling partition (15;31), the device (1) further comprising, within said monolithic structure, means adapted to reduce noisiness, which comprise a shape of the end of the duct (16) for conveying atmospheric air to said bubbling partition (15;31) that lies proximate to the bubbling partition (15;31), the shape of the end of the atmospheric air conveyance duct (16) comprising:
an inclined plane (20) arranged at the rear of the bottom wall of the atmospheric air conveyance duct (16) such that the transverse side (15a) of said bubbling partition (15), at which access of the atmospheric air to said bubbling partition (15) is allowed, is longer than the front side (15b) of said bubbling partition (15), and the holes of the bubbling partition all having the same dimensions; or
the bottom wall (16a) of the duct (16) for conveying the atmospheric air to the bubbling partition (31) is extended in a flat shape to the bottom of the device, so as to allow access of the atmospheric air to said bubbling partition (31) at the front side (31a) thereof, a differentiated calibration of the holes of the bubbling partition (31), with a diameter which increases progressively as one moves away from said front side (3 1a), being provided.

2. The device (1) according to claim 1, **characterized in that** the bubbling partition (15;31) is provided monolithically with the structure of said device.

3. The device (1) according to one or more of the preceding claims, **characterized in that** at the top of the third chamber (300) there is, in said monolithic structure, a separator (23a,23b,24,25a,25b) for the two phases constituted by water and atmospheric air which are mixed together in the stream that exits as a consequence of bubbling from the free surface of the water column (14) that is present in said third chamber (300), with consequent fall of the water onto said column (14) and exit of the atmospheric air from said chamber (300) toward the suction source.

4. The device (1) according to claim 3, **characterized in that** the separator of the two phases constituted by water and atmospheric air comprises, at the top, a baffle (23a) which is adapted to delimit, with an underlying partition (23b), a duct (24) which receives, at the inlet, the stream that comprises said phases mixed together, and has a double outlet port (25a,25b), one of said ports (25a) being adapted to make the water fall back toward the underlying column (14), while the other port (25b) is adapted to send the atmospheric air toward the suction source.

5. The device (1) according to one or more of the preceding claims, **characterized in that** the perforated plug (18) for the inflow of the atmospheric air is provided with more than two holes (18a-d).

6. The device (1) according to one or more of the preceding claims, **characterized in that** the base (14a) of said device, at the column of water (14) that is present in the third chamber (300), is arranged coplanar with the base of the support (22) of said device.

7. The device (1) according to one or more of the preceding claims, **characterized in that** the base plane (28) of a bell (26) comprised within the device (11) for measuring air losses has such an inclination as to make the region that faces directly a port (26a) provided at the lower end of said bell (26) the lowest region.

8. The device (1) according to one or more of the preceding claims, **characterized in that** a duct (9) for connection between the first chamber (100) and the second chamber (200) is provided monolithically with the structure of said device.

9. The device (1) according to one or more of the preceding claims, comprising a tube clamping plate (29) which is adapted to be actuated by an operator between two extreme positions, which correspond to different functional features, **characterized in that** said plate (29) has two regions (29a,29b) with different colors which are designed to be visible selectively in said extreme positions.

10. The device (1) according to one or more of the preceding claims, **characterized in that** a single monolithic structure comprises all the parts which do not move with respect to each other and are potentially uniform in terms of material.

## Patentansprüche

1. Thoraxdrainagevorrichtung (1), die in einer monolithischen Struktur drei miteinander verbundene benachbarte Kammern (100, 200, 300) umfasst, die dazu geeignet sind, in Teilvakuum versetzt zu werden, indem sie mit einer Saugquelle verbunden worden, die in der Lage ist, in der Vorrichtung ein derartiges Vakuum zu erzeugen, das die abgesonderten Flüssigkeiten und die Luftverluste von einem Patienten ableitet, wobei die Kammern umfassen: eine erste Kammer (100) zum Sammeln der abgesonderten Flüssigkeiten, die dazu geeignet ist, mittels eines Schlauchs (6) mit der Brusthöhle eines Patienten verbunden zu werden, um die abgesonderten flüssigkeiten und die Luftverluste, die aus der Brusthöhle austreten, aufzunehmen, eine zweite abdichtende Kammer (200), die am unteren Ende mit einer Vorrichtung (11) zum Messen der Luftverluste ausgestattet ist, eine dritte Kammer (300) zum Einstellen des Vakuums, das in den zwei vorhergehenden Kammern (100, 200) auftritt, umfassend eine Wassersäule (14), durch die atmosphärische Luft geleitet wird, die an dem durch eine perforierte Blasenbildungspartition (15; 31) gebildetcn Boden eintritt und durch einen Kanal (16) dorthin geleitet wird, nachdem sie durch einen geeignet vorgesehenen perforierten Pfropfen (18) eingetreten ist, um durch einen Verbinder (8) abgesaugt zu werden, der zum Anschließen an die Saugquelle ausgebildet ist, wobei die Blasenbildungspartition (15; 31) eine Vorderseite (15b; 31a) und eine Querseite (15a) umfasst, die angrenzend an den Kanal (16) zum Leiten von atmosphärischer Luft zu der Blasenbildungspartition (15; 31) angeordnet ist, wobei die Vorrichtung (1) innerhalb der monolithischen Struktur ferner zum Verringern von Lärm geeignete Mittel umfasst, die eine Form des Endes des Kanals (16) zum Leiten von atmosphärischer Luft zu der Blasonbildungspartition (15; 31) umfassen, das nahe der Blasenbildungspartition (15; 31) liegt, wobei die Form des Endes des Kanals (16) zum Leiten von atmosphärischer Luft umfasst:
eine geneigte Fläche (20), die an der Rückseite der unteren Wand des Kanals (16) zum Leiten von atmosphärischer Luft so angeordnet ist, dass die Querseite (15a) der Blasenbildungspartition (15), an der Zugang der atmosphärischen Luft zu der Blascabildungpartition (15) möglich ist, länger als die Vorderseite (15b) der Blasenbildungspartition (15) ist, und die wobei die Löcher der Blasenbildungspartition alle den gleichen Durchmesser haben; oder
die untere Wand (16a) des Kanals (16) zum Leiten der atmosphärischen Luft zu der Blasenbildungspartition (31) ist in einer geraden Form zum unteren Ende der Vorrichtung verlängert, um den Zugang der atmosphärischen Luft zu der Blasenbildungspartition (31) an deren Vorderseite (31a) zu ermöglichen, wobei eine differenzierte Kalibrierung der Löcher der Blasenbildunspartition (31) mit einem Durchmesser vorliegt, der von der Vorderseite (31 a) weg gehend progressiv zunimmt.

2. Vorrichtung (1) nach Anspruch 1, **dadurch gekennzeichnet, dass** die Blasenbildungspartition (15; 31) monolithisch mit der Struktur der Vorrichtung ausgebildet ist.

3. Vorrichtung (1) nach einem oder mehreren der vorhergehenden Ansprüche, **dadurch gekennzeichnet, dass** am oberen Ende der dritten Kammer (300) in der monolithischen Struktur ein Separator (23a, 23b, 24, 25a, 25b) für die beiden Phasen Wasser und atmosphärische Luft vorliegt, die in dem Strom vermischt sind, der als eine Folge der Blasenbildung von der freien Fläche der Wassersäule (14) in der dritten Kammer (300) austritt, wobei infolgedessen das Wasser auf die Säule (14) fällt und die atmosphärische Luft zu der Saugquelle hin aus der Kammer (300) austritt.

4. Vorrichtung (1) nach Anspruch 3, **dadurch gekennzeichnet, dass** der Separator der beiden Phasen Wasser und atmosphärische Luft an der Oberseite eine Ablenkplatte (23a) umfasst, die dazu geeignet ist, mit einer darunter angeordneten Abtrennung (23b) einen Kanal (24) zu begrenzen, der am Eingang den die vermischten Phasen enthaltenden Strom aufnimmt und der einen doppelten Ausgang (25a, 25b) hat, wobei ein Ausgang (25a) dazu geeignet ist, das Wasser zu der darunter liegenden Säule (14) fallen zu lassen, während der andere Ausgang (25b) dazu geeignet ist, die atmosphärische Luft zu der Saugquelle hin zu lenken,

5. Vorrichtung (1) nach einem oder mehreren der vorhergehenden Ansprüche, **dadurch gekennzeichnet, dass** der perforierte Stopfen (18) für das Einströmen der atmosphärischen Luft mit mehr als zwei Löchern (18a-d) versehen ist.

6. Vorrichtung (1) nach einem oder mehreren der vorhergehenden Ansprüche, **dadurch gekennzeichnet, dass** die Basis (14a) der Vorrichtung an der Wassersäule (14), die in der dritten Kammer (300) vorhanden ist, koplanar mit der Basis der Stützvorrichtung (22) der Vorrichtung angeordnet ist..

7. Vorrichtung (1) nach einem oder mehreren der vorhergehenden Ansprüche, **dadurch gekennzeichnet, dass** die Basisfläche (28) einer Glocke (26), die innerhalb der Vorrichtung (11) zum Messen von Luftverlusten enthalten ist, eine derartige Neigung hat, dass der Bereich, der einem am unteren Ende der Glocke (26) vorgesehenen Ausgang (26a) direkt zugewandt ist, zum tiefsten Bereich wird.

8. Vorrichtung (1) nach einem oder mehreren der vorhergehenden Ansprüche, **dadurch gekennzeichnet, dass** ein Kanal (9) für eine Verbindung zwischen der ersten Kammer (100) und der zweiten Kammer (200) monolithisch mit der Struktur der Vorrichtung vorgesehen ist.

9. vorrichtung (1) nach einem oder mehreren der vorhergehenden Ansprüche, umfassend eine Schlauchklemmplatte (29), die dazu geeignet ist, von einer Bedienperson zwischen zwei Endstellungen betätigt zu werden, die unterschiedlichen Funktionsmerkmalen entsprechen, **dadurch gekennzeichnet, dass** die Platte (29) zwei Bereiche (29a, 29b) mit unterschiedlichen Farben aufweist, die so ausgelegt sind, dass sie in den Endestellungen wahlweise sichtbar sind.

10. Vorrichtung (1) nach einem oder mchrcren der vorhergehenden Ansprüche, **dadurch gekennzeichnet, dass** eine einzige monolithische Struktur alle Teile umfasst, die zueinander unbeweglich und hinsichtlich des Materials potentiell einheitlich sind.

## Revendications

1. Dispositif de drainage thoracique (1), comprenant, dans une structure monolithique, trois chambres contiguës interconnectées (100, 200, 300), qui sont adaptées de façon à êtres mises sous un vide partiel en étant reliées à une source d'aspiration qui est susceptible de créer, à l'intérieur du dispositif, une condition de vide qui éloigne du patient les liquides exsudés et les pertes d'air, lesdites chambres comprenant: une première chambre (100) pour collecter les liquides exsudés, celle-ci étant adaptée de façon à être reliée à l'aide d'un tube (6) à la cavité thoracique d'un patient, de façon à recevoir lesdits liquides exsudés et les pertes d'air qui sortent de ladite cavité, une deuxième chambre d'étanchéité (200), munie en bas d'un dispositif (11) pour mesurer lesdites pertes d'air, une troisième chambre (300) pour ajuster le vide qui se produit à l'intérieur des deux chambres précédentes (100, 200), comprenant une colonne d'eau (14) à travers laquelle on fait passer de l'air atmosphérique qui entre au niveau du fond formé par une séparation à bulles perforée (15; 31), celui-ci y étant convoyé par un conduit (16) après être entré par l'intermédiaire d'un bouchon perforé disposé de façon appropriée (18), afin d'être évacué par l'intermédiaire d'un connecteur (8) qui est conçu de façon à être relié à la source d'aspiration, ladite séparation à bulles (15 ; 31) comprenant un côté avant (15b; 31a), et un côté transversal (15a) qui est disposé au voisinage dudit conduit (16) pour convoyer de l'air atmosphérique vers ladite séparation à bulles (15 ; 31), le dispositif (1) comprenant de plus, à l'intérieur de ladite structure monolithique, des moyens adaptés de façon à réduire le bruit, ceux-ci comprenant une forme de l'extrémité du conduit (16) pour convoyer de l'air atmosphérique vers ladite séparation à bulles (15; 31) qui se trouve à proximité de la séparation à bulles (15; 31), la forme de l'extrémité du conduit de convoyage d'air atmosphérique (16) comprenant :
un plan incliné (20) disposé à l'arrière de la paroi inférieure du conduit de convoyage d'air atmosphérique (16), de telle sorte que le côté transversal (15a) de ladite séparation à bulles (15), où l'accès de l'air atmosphérique à ladite séparation à bulles (15) est autorisé, soit plus long que le côté avant (15b) de ladite séparation à bulles (15), et les trous de la séparation à bulles ayant tous la même dimension ; ou
la paroi inférieure (16a) du conduit (16) pour convoyer l'air atmosphérique vers la séparation à bulles (31) s'étendant sous une forme plate vers le bas du dispositif, de façon à permettre l'accès de l'air atmosphérique à ladite séparation à bulles (31) du côté avant (31a) de celle-ci, une calibration différentiée des trous de la séparation à bulles (31), avec un diamètre qui augmente progressivement en s'éloignant dudit côté avant (31a), étant réalisée.

2. Dispositif (1) selon la revendication 1, **caractérisé en ce que** la séparation à bulles (15 ; 31) est réalisée de façon monolithique avec la structure dudit dispositif.

3. Dispositif (1) selon l'une ou plusieurs des revendications précédentes, **caractérisé en ce qu'**au somment de la troisième chambre (300) se trouve, dans ladite structure monolithique, un séparateur (23a, 23b, 24, 25a, 25b) pour les deux phases constituées par l'eau et l'air atmosphérique qui sont mélangés l'un à l'autre dans le courant qui sort en conséquence de la production de bulles à partir de la surface libre de la colonne d'eau (14) qui est présente dans ladite troisième chambre (300), avec une chute consécutive de l'eau sur ladite colonne (14) et la sortie consécutive de l'air atmosphérique à partir de ladite chambre (300) vers la source d'aspiration.

4. Dispositif (1) selon la revendication 3, **caractérisé en ce que** le séparateur des deux phases constituées par l'eau et l'air atmosphérique comprend, au sommet, un écran (23a) qui est adapté de façon à délimiter, avec une paroi sous-jacente (23b), un conduit (24) qui reçoit, à l'orifice d'entrée, le courant qui comprend lesdites phases mélangées l'une à l'autre, et comporte un double orifice de sortie (25a, 25b), l'un desdits orifices (25a) étant adapté de façon à faire retomber l'eau vers la colonne sous-jacente (14), tandis que l'autre orifice (25b) est adapté de façon à envoyer l'air atmosphérique vers la source d'aspiration.

5. Dispositif (1) selon l'une ou plusieurs des revendications précédentes, **caractérisé en ce que** le bouchon perforé (18) pour l'écoulement d'entrée de l'air atmosphérique est muni de plus de deux trous (18a à d).

6. Dispositif (1) selon l'une, ou plusieurs des revendications précédentes, **caractérisé en ce que** la base (14a) dudit dispositif, au niveau de la colonne d'eau (14) qui est présente dans la troisième chambre (300), est disposée de façon coplanaire à la base du support (22) dudit dispositif.

7. Dispositif (1) selon l'une ou plusieurs des revendications précédentes, **caractérisé en ce que** le plan de base (28) d'une cloche (26) incluse à l'intérieur du dispositif (11) pour mesurer des pertes d'air a une inclinaison telle qu'elle fait de la région qui fait directement face à un orifice (26a) situé à l'extrémité inférieure de ladite cloche (26) la région la plus basse.

8. Dispositif (1) selon l'une ou plusieurs des revendications précédentes, **caractérisé en ce qu'**un conduit (9) pour la liaison entre la première chambre (100) et la deuxième chambre (200) est réalisé de façon monolithique avec la structure dudit dispositif.

9. Dispositif (1) selon l'une ou plusieurs des revendications précédentes, comprenant une plaque de serrage de tube (29) qui est adaptée de façon à être actionnée par un opérateur entre deux positions extrêmes, qui correspondent à des caractéristiques fonctionnelles différentes, **caractérisé en ce que** ladite plaque (29) comporte deux régions (29a, 29b) avec des couleurs différentes qui sont conçues de façon à être visibles de façon sélective dans lesdites positions extrêmes.

10. Dispositif (1) selon l'une ou plusieurs des revendications précédentes, **caractérisé en ce qu'**une structure monolithique unique comprend toutes les parties qui ne se déplacent pas les unes par rapport aux autres et qui sont potentiellement uniformes en termes de matériau.
